(19) Europäisches Patentamt
European Patent Office
Office européen des brevets

(11) Veröffentlichungsnummer: **0 063 738**
**A1**

(12) # EUROPÄISCHE PATENTANMELDUNG


(21) Anmeldenummer: **82103097.0**

(22) Anmeldetag: **10.04.82**

(51) Int. Cl.³: **C 07 C 103/48**, A 01 N 39/02

(30) Priorität: **24.04.81 DE 3116298**

(43) Veröffentlichungstag der Anmeldung: **03.11.82**
**Patentblatt 82/44**

(84) Benannte Vertragsstaaten: **AT BE CH DE FR GB IT LI NL**



(71) Anmelder: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Bergisch-Gladbach 2 (DE)**
Erfinder: **Lürssen, Klaus, Dr., August-Kierspel-Strasse 89, D-5060 Bergisch-Gladbach 2 (DE)**


(54) **N-Phenoxyphenoxyalkyl-carbonylamino-carbonsäure-Derivate, Verfahren zu ihrer Herstellung und ihre Verwendung als Herbizide und Pflanzenwuchsregulatoren.**


(57) Neue N-Phenoxyphenoxyalkyl-carbonyl-amino-carbonsäure-Derivate der Formel

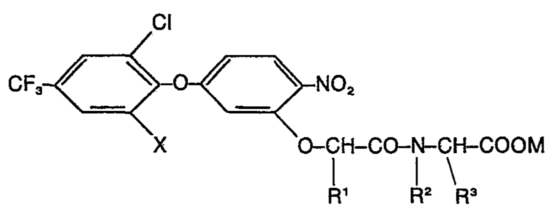

(I)

in welcher

$R^1$, $R^2$ und $R^3$ unabhängig voneinander für Wasserstoff oder Methyl stehen,

X für Wasserstoff oder Chlor steht und

M für Wasserstoff oder ein Metallionenäquivalent steht,

ein Verfahren zur Herstellung der neuen Stoffe sowie deren Verwendung als Herbizide und Pflanzenwuchsregulatoren.


**EP 0 063 738 A1**

0063738

BAYER AKTIENGESELLSCHAFT 5090 Leverkusen, Bayerwerk

Zentralbereich
Patente, Marken und Lizenzen Dü/AB
Ia

N-Phenoxyphenoxyalkyl-carbonylamino-carbonsäure-Derivate, Verfahren zu ihrer Herstellung und ihre Verwendung als Herbizide und Pflanzenwuchsregulatoren

Die Erfindung betrifft neue N-Phenoxyphenoxyalkyl-carbonylamino-carbonsäure-Derivate, ein Verfahren zu deren Herstellung sowie ihre Verwendung als Herbzide und Pflanzenwuchsregulatoren.

Es ist bereits bekannt geworden, daß bestimmte Phenoxyphenoxycarbonsäureamide, wie z.B. 5-(2-Chlor-4-trifluormethyl-phenoxy)-2-nitro-α-phenoxy-propionsäure-3,5-dimethyl-pyrazolid, herbizide Eigenschaften aufweisen (vergl. DE-OS 2 906 237). Die Wirkung der bekannten Verbindungen ist gut, jedoch ist ihre Selektivität nicht immer ausreichend.

Es wurden nun neue N-Phenoxyphenoxyalkyl-carbonylamino-carbonsäure-Derivate der Formel

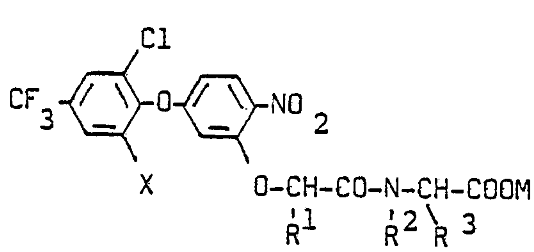

(I)

in welcher

$R^1$, $R^2$ und $R^3$ unabhängig von einander für Wasserstoff oder Methyl stehen,

X für Wasserstoff oder Chlor steht und

M für Wasserstoff oder ein Metallionenäquivalent steht,

gefunden.

Weiterhin wurde gefunden, daß man die N-Phenoxyphenoxyalkylcarbonyl-aminocarbonsäure-Derivate der Formel (I) erhält, wenn man N-Phenoxyphenoxyalkylcarbonyl-aminocarbonsäureester der Formel

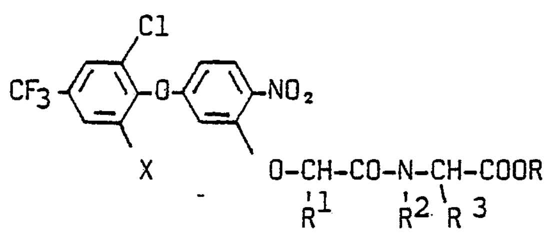

(II)

in welcher

$R^1$, $R^2$, $R^3$ und X die oben angegebene Bedeutung haben und

R für Alkyl mit 1 bis 4 Kohlenstoffatomen steht,

mit wässrigen Lösungen von Alkali- oder Erdalkalihydroxiden gegebenenfalls in Gegenwart von organischen Lösungsmitteln umsetzt und gegebenenfalls anschließend die dabei entstehenden Salze der Formel

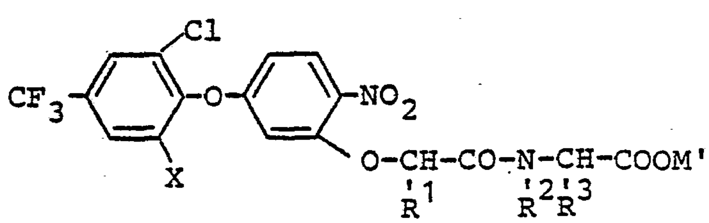

(Ia)

in welcher
$R^1$, $R^2$, $R^3$ und X die oben angegebene Bedeutung haben,
M' für ein Alkalimetall- oder Erdalkalimetall-Ionenäquivalent steht,

durch Behandeln mit Mineralsäure gegebenenfalls in Gegenwart eines Verdünnungsmittels zu den Säuren der Formel

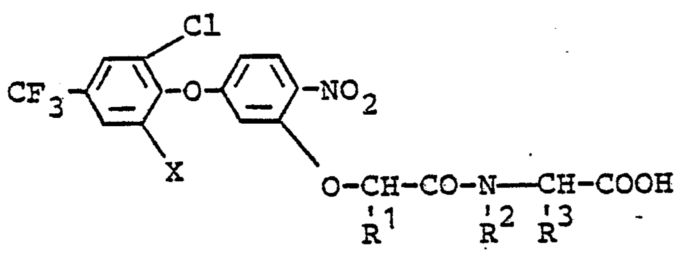

(Ib)

in welcher
$R^1$, $R^2$, $R^3$ und X die oben angegebene Bedeutung haben,

umsetzt und gegebenenfalls diese Säuren durch Behandlung mit Metallhydroxiden oder Metallsalzen in Gegenwart eines Verdünnungsmittels in die jeweiligen Salze überführt.

Schließlich wurde gefunden, daß die neuen N-Phenoxyphenoxyalkyl-carbonylaminocarbonsäure-Derivate der Formel (I) starke herbizide und pflanzenwuchsregulierende Eigenschaften aufweisen.

Überraschenderweise zeigen die erfindungsgemäßen N-Phenoxyphenoxyalkyl-carbonylaminocarbonsäure-Derivate der Formel (I) eine wesentlich bessere herbizide und pflanzenwuchsregulierende Wirksamkeit als aus dem Stand der Technik bekannte Verbindungen analoger Konstitution und gleicher Wirkungsrichtung.

Besonders hervorzuheben ist, daß die erfindungsgemäßen Verbindungen der Formel (I) deutlich bessere selektive herbizide Eigenschaften besitzen als das 5-(2-Chlor-4-trifluormethyl-phenoxy)-2-nitro-α-phenoxy-propionsäure-3,5-dimethyl-pyrazolid, welches ein konstitutionell ähnlicher vorbekannter Wirkstoff gleicher Wirkungsrichtung ist. Die pflanzenwuchsregulierende Wirkung der erfindungsgemäßen Stoffe kann insbesondere in der Anwendung als Baumwolldefoliants genutzt werden.

Die erfindungsgemäßen N-Phenoxyphenoxyalkylcarbonylaminocarbonsäure-Derivate sind durch die Formel (I) allgemein definiert. In dieser Formel stehen vorzugsweise

$R^1$ für Methyl,
$R^2$ für Wasserstoff,
$R^3$ für Wasserstoff
X für Wasserstoff oder Chlor und
M für Wasserstoff, Natrium, Kalium oder ein Magnesium- oder Calcium-äquivalent.

Verwendet man N-(α-(5-(2,6-Dichlor-4-trifluormethyl-phenoxy)-2-nitro-phenoxy)-propionyl)-aminoessigsäuremethylester und Magnesiumhydroxid als Ausgangsstoffe, so kann der Reaktionsablauf nach dem erfindungsgemäßen Verfahren durch das folgende Formelschema wiedergegeben werden:

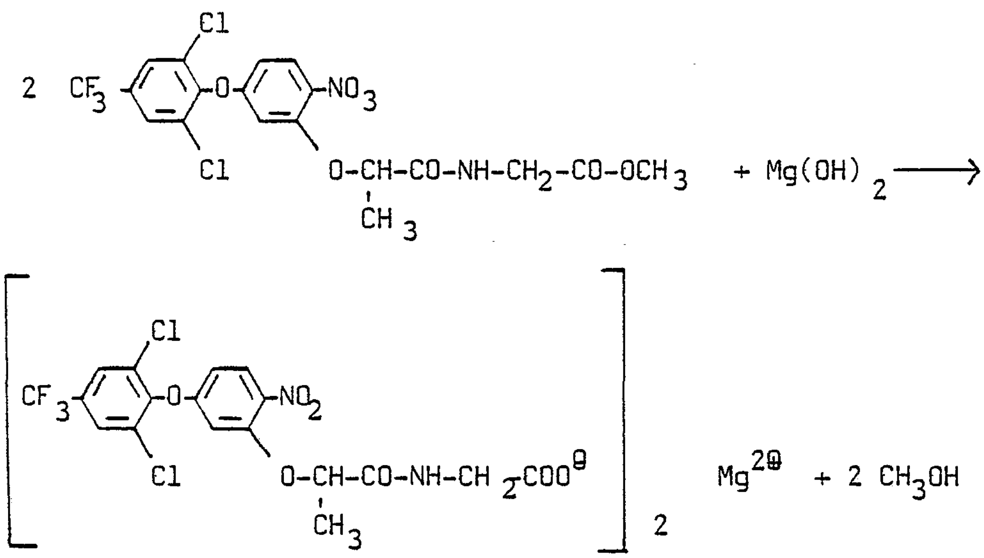

Die bei der Durchführung des erfindungsgemäßen Verfahrens als Ausgangsstoffe benötigten N-Phenoxyphenoxyalkylcarbonylaminocarbonsäureester sind durch Formel (II) definiert. In dieser Formel stehen $R^1$, $R^2$, $R^3$ und X vorzugsweise für diejenigen Reste, die bereits im Zusammenhang mit der Beschreibung der erfindungsgemäßen Stoffe der Formel (I) vorzugsweise für diese Reste genannt wurden.
R steht vorzugsweise für Methyl oder Ethyl.

Als Beispiele für die Verbindungen der Formel (II) seien genannt:
N-(α-(5-(2-Chlor-4-trifluormethyl-phenoxy)-2-nitro-phenoxy)-propionyl)-aminoessigsäuremethylester und -ethylester sowie
N-(α-(5-(2,6-Dichlor-4-trifluormethyl-phenoxy)-2-nitro-phenoxy)-propionyl)-aminoessigsäuremethylester und -ethylester.

Die N-Phenoxyphenoxyalkylcarbonyl-aminocarbonsäureester der Formel (II) sind bekannt oder lassen sich nach üblichen Methoden in einfacher Weise herstellen (vergleiche DE-OS 2 906 237).

So erhält man die Verbindungen der Formel (II) zum Beispiel dadurch, daß man Diphenylether der Formel

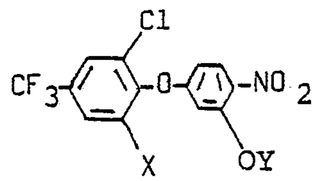

(III)

in welcher

X die oben angegebene Bedeutung hat und

Y für Wasserstoff, Natrium oder Kalium steht,

mit α-Halogen-alkylcarbonyl-aminocarbonsäureestern der Formel

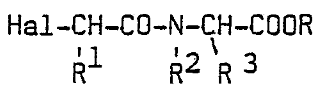

(IV)

in welcher

$R$, $R^1$, $R^2$ und $R^3$ die oben angegebene Bedeutung haben und

Hal für Chlor oder Brom steht,

gegebenenfalls in Gegenwart eines Säurebindemittels, wie z.B. Natriummethylat oder Kaliumcarbonat, und gegebenenfalls in Gegenwart eines polaren Verdünnungsmittels, wie z.B. Methanol, Acetonitril oder Sulfolan, bei Temperaturen zwischen 2o und loo°C umsetzt.

Die Verbindungen der Formeln (III) und (IV) sind bekannt oder lassen sich nach üblichen Methoden in einfacher Weise herstellen.

Als Beispiele für die Verbindungen der Formel (III) seien genannt: 5-(2-Chlor-4-trifluormethyl-phenoxy)-2-nitrophenol und 5-(2,6-Dichlor-4-trifluormethylphenoxy)-2-nitro-phenol sowie deren Natrium- bzw. Kalium-Salze.

Als Beispiele für die Verbindungen der Formel (IV) seien genannt: N-($\alpha$-Chlor-propionyl)-aminoessigsäuremethylester und -ethylester sowie N-($\alpha$-Brom-propionyl)-aminoessigsäuremethylester und -ethylester.

Das erfindungsgemäße Verfahren zur Herstellung der Salze der Formel (Ia) wird unter Verwendung wäßriger Lösungen von Alkali- oder Erdalkalihydroxiden durchgeführt. Vorzugsweise werden Lösungen von Natrium-, Kalium-, Magnesium- oder Calciumhydroxid verwendet.

Vorzugsweise wird das erfindungsgemäße Verfahren zur Herstellung der Salze der Formel (Ia) in Gegenwart von organischen Lösungsmitteln durchgeführt. Als solche kommen insbesondere polare, mit Wasser mischbare Solventien in Frage, wie z.B. Aceton, Acetonitril, Dimethylformamid, Dimethylsulfoxid, Methanol, Ethanol, n- und iso-Propanol.

Die Reaktionstemperaturen können bei der Umsetzung zur Herstellung der Salze der Formel (Ia) innerhalb eines größeren Bereiches variiert werden. Im allgemeinen arbeitet man bei Temperaturen zwischen 0°C und 50°C, vorzugsweise zwischen 15 und 25°C. Das erfindungsgemäße Verfahren wird im allgemeinen unter Normaldruck durchgeführt.

Bei der Durchführung des erfindungsgemäßen Verfahrens

zur Herstellung der Salze der Formel (Ia) setzt man auf 1 Mol N-Phenoxyphenoxyalkylcarbonylaminocarbonsäureester der Formel (II) im allgemeinen 1 bis 2, vorzugsweise 1 bis 1,2 Moläquivalente Alkali- oder Erdalkalihydroxid ein. Im allgemeinen geht man so vor, daß man die Ausgangssubstanz der Formel (II) mit der wäßrigen Lösung eines Alkali- oder Erdalkalihydroxids sowie gegebenenfalls mit einem organischen Lösungsmittel vermischt und das Reaktionsgemisch bei der jeweiligen Temperatur bis zur Beendigung der Umsetzung rührt. Die Isolierung der Stoffe der Formel (Ia) erfolgt nach üblichen Methoden.

Um aus den Salzen der Formel (Ia) die entsprechenden Säuren der Formel (Ib) herzustellen, geht man in der Regel so vor, daß man das zuvor anfallende Reaktionsgemisch ohne vorherige Isolierung der Salze der Formel (Ia) gegebenenfalls nach dem Verdünnen mit Wasser mit Mineralsäure ansäurert. Als Mineralsäuren kommen vorzugsweise wäßrige Salzsäure oder Schwefelsäure in Frage.

Die Reaktionstemperaturen können bei der Herstellung der Säuren der Formel (Ib) aus den Salzen der Formel (Ia) innerhalb eines bestimmten Bereiches variiert werden. Im allgemeinen arbeitet man bei Temperaturen zwischen 0°C und 50°C, vorzugsweise zwischen 15 und 25°C. Im allgemeinen arbeitet man bei dieser Umsetzung unter Normaldruck.

Bei der Herstellung der Säuren der Formel (Ib) behandelt man die Salze der Formel (Ia) mit einem Überschuß

0063738

an Mineralsäure. Die Isolierung der Säuren der Formel (Ib) erfolgt nach üblichen Methoden. Im allgemeinen geht man so vor, daß man das Reaktionsgemisch mit einem mit Wasser praktisch nicht mischbaren organischen Lösungsmittel, wie zum Beispiel Methylenchlorid, extrahiert, die organische Phase mit Wasser wäscht, trocknet und filtriert und das Filtrat unter vermindertem Druck einengt. Die als Rückstand verbleibenden Säuren der Formel (Ib) werden durch ihren Schmelzpunkt charakterisiert.

Um aus den Säuren der Formel (Ib) Salze herzustellen, behandelt man die Säuren in Gegenwart eines Verdünnungsmittels mit Metallhydroxiden oder Metallsalzen. Als Metallhydroxide und Metallsalze kommen vorzugsweise Alkali- und Erdalkali-hydroxide, wie z.B. Natrium-, Kalium-, Magnesium- und Calcium-hydroxid, Alkalialkoholate, wie z.B. Natrium- und Kaliummethylat und -ethylat sowie Alkali- oder Erdalkalichloride, wie z.B. Natrium-, Kalium-, Magnesium- und Calcium-chlorid in Betracht.

Bei der Herstellung von Salzen aus Säuren der Formel (Ib) kommen als Verdünnungsmittel, Wasser, organische Lösungsmittel oder Gemische aus Wasser und organischen Lösungsmitteln in Frage. Als organische Lösungsmittel kommen insbesondere polare, mit Wasser mischbare Solventien in Betracht, wie zum Beispiel Aceton, Acetonitril, Dimethylformamid, Dimethylsulfoxid, Methanol, Ethanol, n- und iso-Propanol.

Die Reaktionstemperaturen können auch bei dieser Umsetzung innerhalb eines bestimmten Bereiches variiert

werden. Im allgemeinen arbeitet man bei Temperaturen zwischen 0°C und 50°C, vorzugsweise zwischen 15°C und 30°C. Im allgemeinen arbeitet man bei dieser Umsetzung unter Normaldruck.

Bei der Herstellung von Salzen aus dem Säuren der Formel (Ib) verfährt man im allgemeinen so, daß man die Säuren der Formel (Ib) mit angenähert äquivalenten Mengen an Metallsalzen im Lösungsmittel vermischt und etwa eine Stunde gut durchrührt. Dann werden die flüchtigen Komponenten unter vermindertem Druck sorgfältig abdestilliert, wobei die Salze der Formel (I) als Rückstand verbleiben.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca,

Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agrophyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können insbesondere zur selektiven Unkrautbekämpfung in Sojabohnen, Baumwolle und Getreidearten eingesetzt werden.

Außerdem besitzen die erfindungsgemäßen Stoffe eine sehr gute pflanzenwuchsregulierende Wirksamkeit. Sie eignen sich besonders gut zur Wuchshemmung, sowie zur Entlaubung und Austrocknung der Blätter bei Baumwolle.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen o,o5 und lo kg Wirkstoff pro ha, vorzugsweise zwischen o,1 und 5 kg/ha.

Herstellung und Verwendung der erfindungsmäßen Wirkstoffe gehen aus den nachfolgenden Beispielen hervor.

Herstellungsbeispiele

Beispiel 1

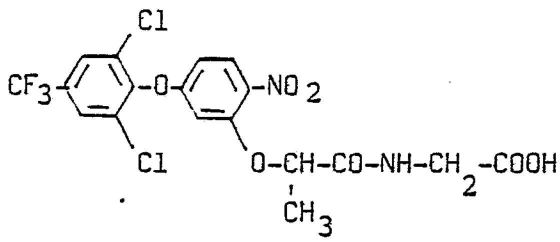

22 g (o,o42 Mol) N-(α-(5-(2,6-Dichlor-4-trifluormethyl-phenoxy)-2-nitro-phenoxy)-propionyl)-aminoessigsäureethylester und 1,8 g (o,o45 Mol) Natriumhydroxid werden mit 25 ml Wasser und 7o ml Acetonitril vermischt und 15o Minuten bei 15-25°C gerührt. Das Gemisch wird dann mit 3oo ml Wasser verdünnt, mit konzentrierter Salzsäure angesäuert und mit 2 x 25o ml Methylenchlorid extrahiert. Die organische Phase wird mit Wasser gewaschen, getrocknet und filtriert. Vom Filtrat wird das Lösungsmittel unter vermindertem Druck sorgfältig abdestilliert. Man erhält 15,8 g (76% der Theorie) N-(α-(5-(2,6-Dichlor-4-trifluormethyl-phenoxy)-2-nitro-phenoxy)-propionyl)-aminoessigsäure als gelben, kristallinen Rückstand vom Schmelzpunkt 73°C.

Beispiel 2

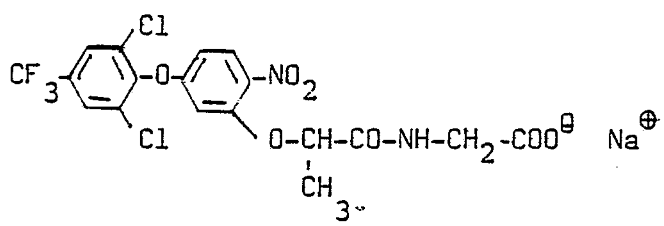

24,9 g (o,o5 Mol) N-(α-(5-(2,6-Dichlor-4-trifluormethyl-phenoxy)-2-nitro-phenoxy)-propionyl)-aminoessigsäure (vergleiche Beispiel 1) werden bei 2o°C zu einer Lösung von 2,8 g Natriummethylat in 8o ml Methanol gegeben, und das Gemisch wird eine Stunde gerührt. Dann wird das Lösungsmittel unter vermindertem Druck sorgfältig abdestilliert. Man erhält 24,9 g (96% der Theorie) N-(α-(5-(2,6-Dichlor-4-trifluormethyl-phenoxy)-2-nitro-phenoxy)-propionyl-aminoessigsäure-Natriumsalz als gelben, kristallinen Rückstand.

Analog Beispiel 1 bzw. Beispiel 2 werden die in der nachstehenden Tabelle aufgeführten Verbindungen der Formel (I) hergestellt:

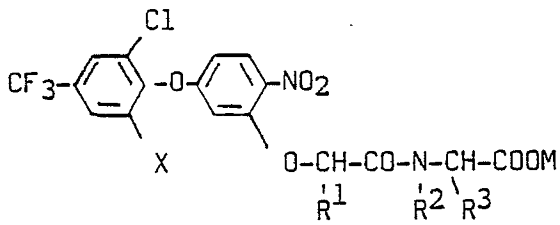

(I)

Tabelle: Beispiele für die Verbindungen der Formel (I)

| Beispiel Nr. | X | $R^1$ | $R^2$ | $R^3$ | M | Schmelzpunkt °C |
|---|---|---|---|---|---|---|
| 3 | H | $CH_3$ | H | H | H | 53 |
| 4 | H | $CH_3$ | H | H | Na | |
| 5 | H | $CH_3$ | H | H | Ca 1/2 | |
| 6 | Cl | $CH_3$ | H | H | Ca 1/2 | |
| 7 | H | $CH_3$ | H | H | K | |
| 8 | Cl | $CH_3$ | H | H | K | |
| 9 | Cl | $CH_3$ | H | H | Mg 1/2 | |
| lo | H | $CH_3$ | H | H | Mg 1/2 | |

In den folgenden Verwendungsbeispielen wird die nachstehend angegebene Verbindung als Vergleichssubstanz eingesetzt:

(A) = 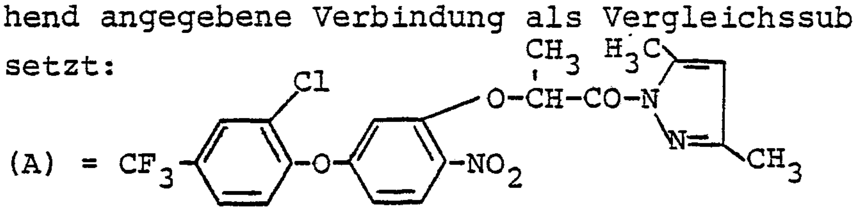

(bekannt aus DE-OS 29 06 237)

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (1), (2), (3), (4) und (5) eine bessere selektive herbizide Wirksamkeit als die Vergleichskomponente (A).

Beispiel B

Entlaubung und Austrocknung der Blätter bei Baumwolle

Lösungsmittel: 3o Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die erfindungsgemäßen Wirkstoffe (3), (4) und (5) eine starke bzw. sehr starke Wirkung.

0063738

Patentansprüche

1. N-Phenoxyphenoxyalkyl-carbonylaminocarbonsäure-Derivate der Formel

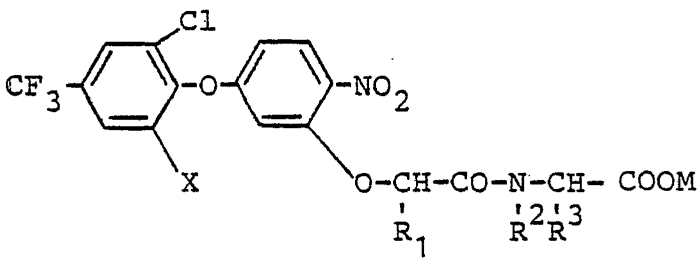

(I)

in welcher
$R^1$, $R^2$ und $R^3$ unabhängig von einander für Wasserstoff oder Methyl stehen,
X für Wasserstoff oder Chlor und
M für Wasserstoff oder ein Metallionenäquivalent steht.

2. Verfahren zur Herstellung von N-Phenoxyphenoxyalkyl-carbonylamino-carbonsäure-Derivaten der Formel

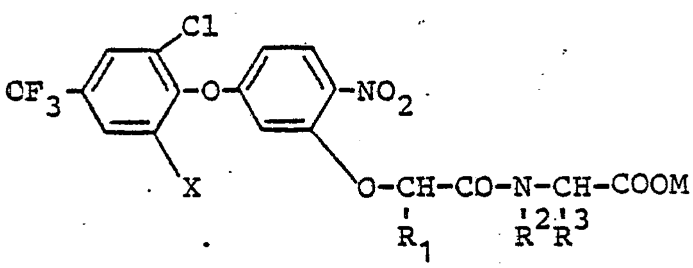

(I)

in welcher
$R^1$, $R^2$ und $R^3$ unabhängig von einander für Wasserstoff oder Methyl stehen,

X für Wasserstoff oder Chlor steht und

M für Wasserstoff oder ein Metallionenäquivalent steht,

dadurch gekennzeichnet, daß man N-Phenoxyphenoxyalkylcarbonyl-aminocarbonsäure-ester der Formel

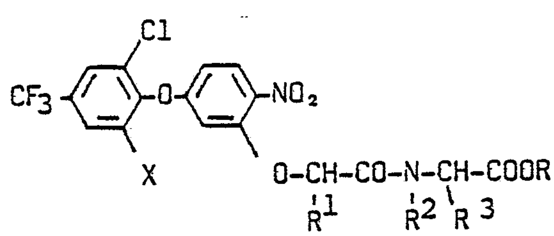

(II)

in welcher

$R^1$, $R^2$, $R^3$ und X die oben angegebene Bedeutung haben und

R für Alkyl mit 1 bis 4 Kohlenstoffatomen steht,

mit wäßrigen Lösungen von Alkali- oder Erdalkalihydroxiden gegebenenfalls in Gegenwart von organischen Lösungsmitteln umsetzt und gegebenenfalls anschließend die dabei entstehenden Salze der Fomel

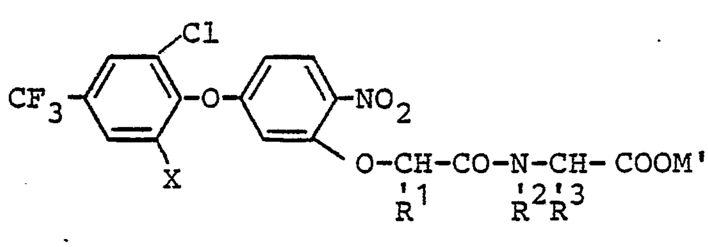

(Ia)

in welcher

0063738

$R^1$, $R^2$, $R^3$ und X die oben angegebene Bedeutung haben,
M' für ein Alkalimetall- oder Erdalkalimetall-Ionenqäuivalent steht,

durch Behandeln mit Mineralsäure gegebenenfalls in Gegenwart eines Verdünnungsmittels zu den Säuren der Formel

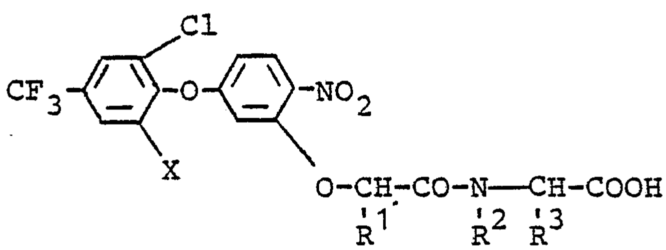

(Ib)

in welcher
$R^1$, $R^2$, $R^3$ und X die oben angegebene Bedeutung haben,

umsetzt und gegebenenfalls diese Säuren durch Behandlung mit Metallhydroxiden oder Metallsalzen in Gegenwart eines Verdünnungsmittels in die jeweiligen Salze überführt.

3. Herbizide und pflanzenwuchsregulierende Mittel, gekennzeichnet durch einen Gehalt an mindestens einem N-Phenoxyphenoxyalkyl-carbonylamino-carbonsäure-Derivat der Formel I.

4. Verfahren zur Bekämpfung von Unkräutern sowie zur Regulierung des Pflanzenwachstums, dadurch gekennzeichnet, daß man N-Phenoxyphenoxyalkyl-carbonylaminocarbonsäure-Derivate der Formel (I) auf die Pflanzen und/oder deren Lebensraum ausbringt.

5. Verwendung von N-Phenoxyphenoxyalkyl-carbonylamino-carbonsäure-Derivaten der Formel (I) zur Bekämpfung von Unkräutern sowie zur Regulierung des Pflanzenwachstums.

6. Verfahren zur Herstellung von herbiziden bzw. pflanzenwuchsregulierenden Mitteln, dadurch gekennzeichnet, daß man N-Phenoxyphenoxyalkyl-carbonylamino-carbonsäure-Derivate der Formel (I) mit Streckmitteln und/oder oberflächenaktiven Stoffen vermischt.

7. N-Phenoxyphenoxyalkyl-carbonylamino-carbonsäure-Derivat der Formel

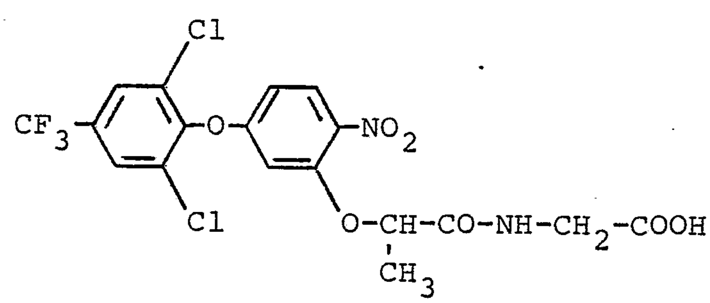

8. N-Phenoxyphenoxyalkyl-carbonylamino-carbonsäure-Derivat der Formel

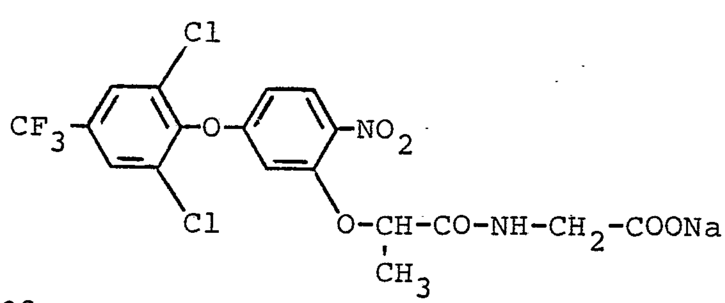

9. N-Phenoxyphenoxyalkyl-carbonylamino-carbonsäure-Derivat der Formel

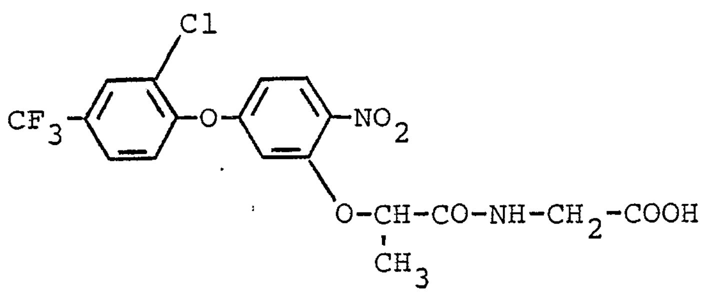

Europäisches Patentamt

# EUROPÄISCHER RECHERCHENBERICHT

0063738

Nummer der Anmeldung

EP 82 10 3097

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 014 900 (BAYER)<br>* Anspruche *<br>--- | 1,3-6 | C 07 C 103/48<br>A 01 N 39/02 |
| D,Y | EP-A-0 014 880 (BAYER)<br>* Anspruche *<br>--- | 1,3-6 | |
| P,Y | DE-A-2 948 095 (HOECHST)<br>* Anspruche *<br>----- | 1,3-6 | |
| | | | RECHERCHIERTE SACHGEBIETE (Int. Cl. 3) |
| | | | C 07 C 103/00<br>A 01 N 39/00 |

Der vorliegende Recherchenbericht wurde für alle Patentansprüche erstellt.

| Recherchenort | Abschlußdatum der Recherche | Prüfer |
|---|---|---|
| DEN HAAG | 23-06-1982 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82